# EUROPEAN PATENT SPECIFICATION

(11) **EP 1 633 369 B1**
(45) Date of publication and mention of the grant of the patent: **18.04.2007**
(21) Application number: 03811569.7
(22) Date of filing: 13.11.2003
(51) Int. Cl.: A61K 31/575, A61K 31/167, A61K 9/72, A61J 3/00, A61P 11/06

(54) **PROCESS TO MINIMIZE LOSS THROUGH ADHESION TO EQUIPMENT SURFACES**
VERFAHREN ZUR MINIMIERUNG VON VERLUSTEN DURCH ADHÄSION AN GERÄTEOBERFLÄCHEN
PROCEDE DE REDUCTION DE PERTES PAR ADHESION A DES SURFACES D'EQUIPEMENT

(30) Priority: 15.11.2002 SE 0203376
(43) Date of publication of application: 15.03.2006
(73) Proprietor: AstraZeneca AB, 151 85 Södertälje (SE)
(72) Inventor: Cawthorne, Simon, 3M Healthcare, Loughborough, Leicestershire LE11 1EP (GB)
(86) International application number: PCT/SE2003/001760
(87) International publication number: WO 2004/045621

(56) References cited:
- WO-A1-00/53188
- WO-A1-01/78737
- WO-A1-02/03958
- WO-A1-93/11773
- US-A- 5 972 919
- US-A- 6 004 537

## Description

The present invention relates to a new process for the preparation of combinations of drugs, in particular combinations of inhaled corticosteroids and β2 agonists.

It is known that some pharmaceutical compounds suitable for administration via pressurised metered dose inhalers (pMDI's) have a tendency to adhere to the container surface. This problem is particularly noticeable with HFA formulations and there are patent applications, which detail the problem and possible solutions. For example, EP642992, WO96/32099 and US6253762 deal with adhesion on the walls of the MDI canister, but the term container used above is not limited to only the canister.

Less well known is that fact that the problem also applies to the surfaces of the manufacturing vessel and filling equipment. This makes filling of the metered dose inhaler container and subsequent dosing from those containers, less uniform. The problem has traditionally been overcome by adding an overage to the formulation, i.e. adding more of one or more of the ingredients than is theoretically required. However the effect cannot always be consistent enough for the overage method to alleviate the problem and as the extent of adhesion is not constant from batch to batch, much validation work has to be carried out to define the range of permitted overage for that product which will most likely result in an acceptable product in terms of reliable and consistent drug content for each dose. Details of such an investigation can be found in the proceedings of *Drug delivery to the Lungs 1997 pgs47-50 "Production Scale Optimisation of the Manufacturing Process for a HFA 134A Metered Dose Inhaler Containing Both Salmeterol and Fluticasone Propionate" Duquemin et al.* One of the parameters to be optimised was the drug overage I.e the amount of extra drug added to compensate for the loss of drug during the production process.
In the case of expensive drug compound, drug overages can add significantly to the cost of the final product. Also this is not nessessarily an easy solution as each active has to be optimised in combination products and the adhesion as mentioned above can vary from vessel to vessel and batch to batch. This can result in unacceptable rate of batch failure on dose content from the MDIs produced

Another factor is that the permitted overages are limited by the regulatory bodies to less than 10% of actives, which for a dilute product in a large production vessel may not be enough to cover losses.

It was found that if the actives were added to the mix in a predetermined and precise stepwise manner, one active added first in preference to the other, then no overages were necessary. This is a significant and suprising finding.

It would be assumed that as part of the mixing process, over time, the adhesion of each drug would be in proportion to a) its content in the mix and b) the area of the vessel. This has not turned out to be the case as no overage is required even in a large production vessel.

The first aspect of the invention therefore provides a process for adding components to a multicomponent pharmaceutical suspension formulation for inhalation comprising a step wise addition of the components to a container wherein the component of largest mass is added first in order to minimize or iradicate losses due to adhesion of component to the container surface..

The invention also provides a process for adding components to a multicomponent pharmaceutical formulation for inhalation comprising a step wise addition of the components to a container in order to minimize or iradicate losses due to adhesion of component to the container surface. The term "container" means any vessel suitable such as a glass or metal vessel used for the preparation of a pharmaceutical formulation such as pilot plant vessels and larger vessels used for pharmaceutical manufacture.

This invention particularly applies to formulations where the drug or drugs have a tendency to adhere to the surfaces of the manufacturing equipment in which they are mixed and through which they are dispensed into the container, canister or dosing device.

Also this invention particularly applies to the manufacture of suspension formulations and most preferably to that of drug suspended in a hydrofluoroalkane propellant. Preferred propellants are HFA 134a and HFA 227 and mixtures thereof.

The methodology of the production is known in the art and comprises the usual manufacturing equipment for preparation of large quantities of suspension formulation under pressure, for through-the-valve pressure filling of the canisters. However the principal could equally apply to equipment of smaller surface area and/or sytems not under pressure, for instance when MDIs are produced under the method of cold fill or where aqueous suspension formulations are being prepared.

Preferably the drugs of the invention are budesonide and formoterol or a hydrate of a salt thereof, in particular formoterol fumarate dihydrate.

Essentially, the larger component is added first. Essentially, therefore, the budesonide is added prior to the addition of the formoterol fumarate dihydrate.

The process of controlled addition significantly reduces the Formoterol normally lost during manufacture such that the manufacturing overages are significantly reduced, or preferably, not required at all.

Suitably the molar ratio of the budesonide to the formoterol fumarate dihydrate is from 2500:1 to 1:1.

The molar ratio of the budesonide to the formoterol fumarate dihydrate is preferably from 555:1 to 1:1, and more preferably from 150:1 to 1:1. The molar ratio of the budesonide to the formoterol fumarate dihydrate is even more preferably from 133:1 to 6:1. The molar ratio of the budesonide to the formoterol fumarate dihydrate is most preferably 50:1 to 6:1

In terms of dosing ratios for a combination product producted by this method, ratios in weight/weight terms are: Budesonide to Formoterol of 200:1 to 1:1, or more preferably 40:1 to 10:1.

In a further aspect the invention provides a pharmaceutical formulation prepared according to the above process and the use of such a formulation in therapy, in particular for asthma and COPD.

The formulations prepared according to the invention optionally comprise one or more pharmaceutically acceptable suspending agents, valve lubricants, flavourings, bulking agents, sweetners or cosolvents. The formulations are preferably in the form of a micronised powder for inhalation suspended in a hydrofluoroalkane propellant, wherein the particles of the pharmaceutically active ingredients have a mass median diameter of less than 10 µm.

### EXPERIMENTAL DETAILS

The invention is illustrated by the following examples.

The examples shown below in Table 1 are of nine batches produced at a commercial manufacturing site

The amount of the active ingredients in the resulting pMDI's was assayed and compared to theoretical. The resulting API (active pharmaceutical ingredient) concentration was determined by Total Can Assay. The differences seen cannot be attributed to error in assay results due to analytical variation (Assay RSD 0.9%)

The results for each batch show the overage of Formoterol added, the theoretical amount expected if no loss occurs, the resulting assay figure and which API was added first.

**TABLE 1**

| Batch | % Overage FFD | Order of Addition | Theoretical FFD % w/w | Actual FFD % w/w found on assay | % Difference between theoretical and actual | Mean of test results % |
|---|---|---|---|---|---|---|
| 1 | 4.3 | FFD 1^{st} | 0.0073 | 0.0071 | 2.7 % | |
| 2 | 4.3 | FFD 1^{st} | 0.0073 | 0.0070 | 4.1 % | |
| 3 | 0 | FFD 1^{st} | 0.0069 | 0.0067 | 2.9 % | 3.23 |
| 4 | 3.4 | BUD 1^{st} | 0.0072 | 0.0071 | 1.4% | |
| 5 | 0 | BUD 1^{st} | 0.0069 | 0.0068 | 1.4 % | |
| 6 | 0 | BUD 1^{st} | 0.0069 | 0.0067 | 2.9 % | |
| 7 | 0 | BUD 1^{st} | 0.0069 | 0.0068 | 1.4 % | |
| 8 | 0 | BUD 1^{st} | 0.0069 | 0.0067 | 2.9 % | |
| 9 | 0 | BUD 1^{st} | 0.0069 | 0.0068 | 1.4 % | 1.9 |

| | | | | | | |
|---|---|---|---|---|---|---|
| FFD = Formoterol fumurate dihydrate BUD = budesonide | | | | | | |

### Conclusion

The addition of Budesonide prior to the addition of Formoterol
1 reduces the amount of Formoterol that is lost during manufacture
2 such that the manufacturing overages are significantly reduced or negated entirely
3 gives advantage over random addition in that this method makes the outcome of the final API concentration more consistent and well within manufacturing allowances (+/- 5%)
4 reduces the likelihood of batch failure
5 thereby reducing overall costs.

## Claims

**1.** A process for adding components to a multicomponent pharmaceutical suspension formulation for inhalation comprising a step wise addition of the components to a container wherein the component of largest mass is added first in order to minimize or iradicate losses due to adhesion of component to the container surface.

**2.** A process according to claim 1 in which the formulation components are active drug substances.

**3.** A process according to claim 1 or 2 in which the formulation is for a pressurized metered dose inhaler.

**4.** A process according to any one of claims 1 to 3 in which the active drug substances are budesonide and formoterol, a pharmaceutically acceptable salt or solvate thereof, or a solvate of such a salt.

**5.** A process according to any one of claims 2 to 5 in which the active drug substances are budesonide and formoterol fumarate dihydrate.

**6.** A process according to claim 5 in which the molar ratio of the budesonide to the formoterol fumarate dihydrate is from 2500:1 to 1:1.

**7.** A pharmaceutical formulation prepared according to the process defined in any one of claims 1 to 6.

**8.** A formulation according to claim 7 in which the drugs are suspended in one or more HFA propellants.

**9.** A formulation according to claim 8 in which the HFA propellants are HFA 134a or HFA 227 or a mixture thereof.

**9.** A formulation according to claim 8 in which the drugs are suspended in one or more HFA propellants.

**10.** A formulation according to claim 9 in which the HFA propellants are HFA 134a or HFA 227 or a mixture thereof.

## Patentansprüche

1. Verfahren zum Versetzen einer mehrkomponentigen pharmazeutischen Suspensionsformulierung zur Inhalation mit Komponenten, die schrittweise in ein Behältnis eingebracht werden, wobei die Komponente mit der größten Masse zuerst zugesetzt wird, um Verluste aufgrund von Adhäsion der Komponente an der Behältnisoberfläche zu minimieren oder gänzlich zu vermeiden.

2. Verfahren nach Anspruch 1, wobei es sich bei den Formulierungskomponenten um Arzneimittelwirkstoffe handelt.

3. Verfahren nach Anspruch 1 oder 2, wobei die Formulierung für ein unter Druck stehendes Inhalationsgerät zur dosierten Verabreichung gedacht ist.

4. Verfahren nach einem der Ansprüche 1 bis 3, wobei es sich bei den Arzneimittelwirkstoffen um Budesonid und Formoterol, ein pharmazeutisch annehmbares Salz oder Solvat davon oder ein Solvat eines derartigen Salzes handelt.

5. Verfahren nach einem der Ansprüche 2 bis 5, wobei es sich bei den Arzneimittelwirkstoffen um Budesonid und Formoterolfumaratdihydrat handelt.

6. Verfahren nach Anspruch 5, wobei das Molverhältnis von Budesonid zu Formoterolfumaratdihydrat von 2500:1 bis 1:1 beträgt.

7. Pharmazeutische Formulierung, hergestellt nach dem in einem der Ansprüche 1 bis 6 definierten Verfahren.

8. Pharmazeutische Formulierung nach Anspruch 7, bei der die Arzneimittel in einem oder mehreren HFA-Treibgasen suspendiert sind.

9. Pharmazeutische Formulierung nach Anspruch 8, bei der es sich bei den HFA-Treibgasen um HFA 134a oder HFA 227 oder um ein Gemisch davon handelt.

## Revendications

1. Procédé d'addition de composants à une formulation pharmaceutique en suspension à plusieurs composants pour inhalation, comprenant une addition par étapes des composants dans un récipient, **caractérisé en ce que** le composant ayant la masse la plus élevée est ajouté en premier afin de minimiser ou de supprimer les pertes dues à l'adhérence du composant à la surface du récipient.

2. Procédé selon la revendication 1, dans lequel les composants de la formulation sont des substances médicamenteuses actives.

3. Procédé selon la revendication 1 ou 2, dans lequel la formulation est destinée à un inhalateur doseur sous pression.

4. Procédé selon l'une quelconque des revendications 1 à 3, dans lequel les substances médicamenteuses actives sont le budésonide et le formotérol, un sel ou solvate pharmaceutiquement acceptable de ceux-ci, ou un solvate d'un tel sel.

5. Procédé selon l'une quelconque des revendications 2 à 5, dans lequel les substances médicamenteuses actives sont le budésonide et le fumarate de formotérol dihydraté.

6. Procédé selon la revendication 5, dans lequel le rapport molaire du budésonide au fumarate de formotérol dihydraté va de 2500:1 à 1:1.

7. Formulation pharmaceutique préparée conformément au procédé défini dans l'une quelconque des revendications 1 à 6.

8. Formulation selon la revendication 7, dans laquelle les médicaments sont mis en suspension dans un ou plusieurs agents propulseurs HFA.

9. Formulation selon la revendication 8, dans laquelle les agents propulseurs HFA sont le HFA 134a ou le HFA 227 ou un mélange de ceux-ci.
